# EUROPEAN PATENT APPLICATION

(11) **EP 1 157 739 A1**
(43) Date of publication of application: **28.11.2001**
(21) Application number: 99951217.1
(22) Date of filing: 02.11.1999
(51) Int. Cl.: B01J 23/54, C07C 53/08, C07C 51/25

(54) **CATALYST FOR PRODUCING ACETIC ACID, METHOD FOR PREPARING THE SAME AND METHOD FOR PRODUCING ACETIC ACID USING THE SAME**

(30) Priority: 26.02.1999 JP 4974099; 03.03.1999 JP 5533099; 14.04.1999 JP 10636099
(71) Applicant: SHOWA DENKO KABUSHIKI KAISHA, Tokyo 105-8518 (JP)
(72) Inventor: HIGASHI, Tomoyoshi, Oita Works, Showa Denko K. K., Oita-shi, Oita 870-0111 (JP); ABE, Kenichi, Oita Works, Showa Denko K. K., Oita-shi, Oita 870-0111 (JP); YAMADA, Kenji, Oita Works, Showa Denko K. K., Oita-shi, Oita 870-0111 (JP); OGUCHI, Wataru, Oita Works, Showa Denko K. K., Oita-shi, Oita 870-0111 (JP); UCHIDA, Hiroshi, Oita Works, Showa Denko K. K., Oita-shi, Oita 870-0111 (JP); KADOWAKI, Yasushi, Oita Works, Showa Denko K. K., Oita-shi, Oita 870-0111 (JP)
(74) Representative: Strehl Schübel-Hopf & Partner
(86) International application number: JP9906117
(87) International publication number: WO0051725

(57) **Abstract**

Catalysts for production of acetic acid which comprise metallic palladium, metallic gold and heteropoly acids and/or salts thereof as essential components, and having the metallic palladium and metallic gold present in a specific weight ratio. Catalysts for production of acetic acid containing metallic palladium and heteropoly acids and/or their salts as essential components, in which the heteropoly acid salts comprise a metal salt wherein all or a portion of the hydrogen atoms of the heteropoly acid are replaced with at least one element selected from the group consisting of Group 6 elements, Group 7 elements, Group 9 elements and Group 10 elements of the Periodic Table.

## Description

### Cross-Reference to Related Applications

This application is an application filed under 35 U.S.C. §111(a) claiming benefit pursuant to 35 U.S.C. §119(e)(1) of the filing date of the Provisional Application 60/128,418 filed April 8, 1999, and the Provisional Application 60/134,942 filed May 19, 1999, pursuant to 35 U.S.C. §111(b).

### Technical Field

The present invention relates to a catalyst used when producing acetic acid from ethylene and oxygen by a one-stage catalytic reaction, to a process for production of the catalyst and to a process for production of acetic acid using the catalyst.

### Background Art

Acetic acid production processes according to the prior art that have been realized include a process by oxidation of acetaldehyde, a process by reaction of methanol and carbon monoxide and a process by oxidation of lower hydrocarbons.

The process by oxidation of acetaldehyde (see Kagaku Daijiten 3, Kyoritsu Publishing, 8/5/1969, 9th Edition, "Acetic acid") is a two-stage process wherein acetic acid is produced from ethylene through an acetaldehyde intermediate. However, since the palladium ion which contributes to oxidation of ethylene cannot oxidize the acetaldehyde produced, direct synthesis of acetic acid is difficult by this process. The process for reaction of methanol and carbon monoxide (Japanese Unexamined Patent Publication No. 60-54334 and No. 60-239434) has a problem in that the rhodium used as the catalyst is extremely expensive.

On the other hand, the process by oxidation of lower hydrocarbons (Japanese Unexamined Patent Publication No. 5-246934 and No. 5-178785) allows synthesis of acetic acid by a single stage. However, because of the relatively stringent reaction conditions there are problems of abundant by-products and difficulty in improving the reaction selectivity and yield.

Processes for production of acetic acid from ethylene by one-stage oxidation have several advantages in terms of industrial production steps and cost, and a number of such processes have therefore been proposed as alternatives to the conventional processes. For example, there has been proposed a liquid phase one-stage oxidation process using oxidation-reduction catalysts of metal ion pairs such as palladium-cobalt, iron, etc. (French Patent Publication No. 1,448,361), as well as gas phase one-stage oxidation processes using catalysts comprising palladium-phosphoric acid or sulfur-containing modifiers (Japanese Unexamined Patent Publication No. 47-13221 and No. 51-29425), catalysts comprising palladium salts of certain heteropoly acids (Japanese Unexamined Patent Publication No. 54-57488) and catalysts comprising ternary oxygen compounds (Japanese Examined Patent Publication No. 46-6763). However, not all acetic acid production processes employing these catalysts can be said to give sufficient performance when carried out on an industrial scale.

More recently there has been proposed a process for production of acetic acid from ethylene and oxygen by a gas phase one-stage reaction, using a catalyst containing metallic palladium and at least one compound selected from among heteropoly acids and/or their salts (Japanese Unexamined Patent Publication No. 7-89896 and No. 9-67298). According to the process using this catalyst it is possible to obtain acetic acid at a relatively high yield and in a highly selective manner. However, a higher performance catalyst is desired for production on an industrial scale.

As regards the catalysts for production of acetic acid disclosed in Japanese Unexamined Patent Publication No. 7-89896 and Japanese Unexamined Patent Publication No. 9-67298 cited above as prior art, Group 11 elements including gold are indicated as constituent elements of the catalysts under the claims and the Detailed Description of the Invention, but they nowhere mention the effectiveness of using a catalyst containing metallic gold and metallic palladium in a specific proportion, nor do the examples present any catalyst for production of acetic acid which contains metallic gold.

### Disclosure of the Invention

It is an object of the present invention to provide a higher performance catalyst to be used in a process for synthesis of acetic acid from ethylene and oxygen by a gas phase one-stage reaction with a catalyst containing metallic palladium and at least one compound selected from among heteropoly acids and salts thereof, to provide a process for production of the catalyst and to provide a process for production of acetic acid using the catalyst.

In order to achieve the object stated above, the present inventors have carried out diligent research aimed at increasing the performance of catalysts used for synthesis of acetic acid from ethylene and oxygen by gas phase one-stage reactions. As a result, the inventors have completed the present invention upon first finding that, in a process for production of acetic acid by reacting ethylene and oxygen in a gas phase in the presence of a catalyst having (a) metallic palladium, (b) metallic gold and (c) at least one compound selected from among heteropoly acids and/or salts thereof loaded on a carrier, the space time yields of acetic acid are drastically improved when the weight ratio of (a) metallic palladium and (b) metallic gold in the catalyst is a specific proportion.

Specifically, the invention (I) provides a catalyst for production of acetic acid, which is a catalyst used in a process for production of acetic acid that comprises reacting ethylene and oxygen in a gas phase and having (a) metallic palladium, (b) metallic gold and (c) at least one compound selected from among heteropoly acids and/or salts thereof loaded on a carrier, wherein the weight ratio of the (a) metallic palladium and (b) metallic gold is in the range of (a) metallic palladium:(b) metallic gold = 1:0.1-5.0.

The invention (II) provides a catalyst for production of acetic acid according to the invention (I), wherein (d) at least one element selected from the group consisting of Group 6 elements, Group 7 elements, Group 8 elements, Group 9 elements, Group 10 elements and Group 12 elements of the Periodic Table (in conformity with the Revised Standards for International Union of Pure and Applied Chemistry Inorganic Chemical Nomenclature (1989), same hereunder) is also loaded on the carrier in addition to the (a) metallic palladium, (b) metallic gold and (c) at least one compound selected from among heteropoly acids and/or salts thereof.

The invention (III) provides a catalyst for production of acetic acid according to the invention (I), wherein (e) at least one element selected from the group consisting of Group 11 elements, Group 14 elements, Group 15 elements and Group 16 elements of the Periodic Table is also loaded on the carrier in addition to the (a) metallic palladium, (b) metallic gold and (c) at least one compound selected from among heteropoly acids and/or salts thereof.

The invention (IV) provides a catalyst for production of acetic acid according to the invention (I), wherein (d) at least one element selected from the group consisting of Group 6 elements, Group 7 elements, Group 8 elements, Group 9 elements, Group 10 elements and Group 12 elements of the Periodic Table and (e) at least one element selected from the group consisting of Group 11 elements, Group 14 elements, Group 15 elements and Group 16 elements of the Periodic Table are also loaded on the carrier in addition to the (a) metallic palladium, (b) metallic gold and (c) at least one compound selected from among heteropoly acids and/or salts thereof.

The invention (V) provides a process for production of the aforementioned catalyst for production of acetic acid.

The invention (VI) provides a process for production of acetic acid whereby acetic acid is synthesized from ethylene and oxygen by a gas phase one-stage reaction, using the aforementioned catalyst for production of acetic acid.

The present inventors have also completed the present invention upon discovering a catalyst which gives higher space time yields, has lower carbon dioxide selectivity and undergoes less deterioration compared to conventional processes, by using a metal salt wherein all or a portion of the hydrogen atoms of the heteropoly acid in the metallic palladium-heteropoly acid catalyst are replaced with at least one element selected from the group consisting of Group 6 elements, Group 7 elements, Group 9 elements and Group 10 elements of the Periodic Table.

Specifically, the invention (VII) provides a catalyst for production of acetic acid, which is a catalyst that is used in a process for production of acetic acid by reacting ethylene and oxygen and contains (a) metallic palladium and (b) a heteropoly acid salt, wherein the (b) heteropoly acid salt comprises a metal salt wherein all or a portion of the hydrogen atoms of the heteropoly acid are replaced with at least one element selected from the group consisting of Group 6 elements, Group 7 elements, Group 9 elements and Group 10 elements of the Periodic Table, or the same catalyst loaded on a carrier.

The invention (VIII) provides a catalyst for production of acetic acid, which is a catalyst that is used in a process for production of acetic acid by reacting ethylene and oxygen and contains (a) metallic palladium, (b) a heteropoly acid salt and (c) at least one element selected from the group consisting of Group 8 elements, Group 11 elements, Group 12 elements, Group 14 elements, Group 15 elements and Group 16 elements of the Periodic Table, wherein the (b) heteropoly acid salt comprises a metal salt wherein all or a portion of the hydrogen atoms of the heteropoly acid are replaced with at least one element selected from the group consisting of Group 6 elements, Group 7 elements, Group 9 elements and Group 10 elements of the Periodic Table, or the same catalyst loaded on a carrier.

The invention (IX) provides a process for production of the aforementioned catalyst for production of acetic acid.

The invention (X) provides a process for production of the aforementioned catalyst for production of acetic acid, which is loaded on a carrier.

The invention (XI) provides a process for production of acetic acid whereby acetic acid is synthesized from ethylene and oxygen by a gas phase one-stage reaction using the aforementioned catalyst for production of acetic acid.

### Best Mode for Carrying Out the Invention

The present invention will now be explained in more detail.

The catalyst for production of acetic acid according to the invention (I) is a catalyst used in a process for production of acetic acid that comprises reacting ethylene and oxygen in a gas phase and having (a) metallic palladium, (b) metallic gold and (c) at least one compound selected from among heteropoly acids and/or salts thereof loaded on a carrier, wherein the weight ratio of the (a) metallic palladium and (b) metallic gold is in the range of (a) metallic palladium:(b) metallic gold = 1:0.1-5.0.

The palladium contained in the catalyst according to the invention (I) is metallic palladium, and the gold is metallic gold. There are no particular restrictions on the heteropoly acid, and it may be a heteropoly acid containing phosphorus, silicon, boron, aluminum, germanium, titanium, zirconium, cerium, cobalt or chromium as the hetero atom and at least one element selected from the group consisting of molybdenum, tungsten, vanadium, niobium and tantalum as the polyatom. As specifically preferred ones there may be mentioned tungstosilicic acid, tungstophosphoric acid, molybdosilicic acid, molybdophosphoric acid, molybdotungstophosphoric acid, molybdotungstosilicic acid, vanadotungstophosphoric acid, vanadotungstosilicic acid, vanadomolybdosilicic acid, tungstoboric acid, molybdoboric acid and molybdotungstoboric acid. Particularly preferred among these are heteropoly acids wherein the hetero atom is phosphorus or silicon and the polyatom is at least one element selected from the group consisting of tungsten, molybdenum and vanadium.

The salt of the heteropoly acid is a metal salt or onium salt wherein all or a portion of the hydrogen atoms of the acid produced by condensation of the two or more different inorganic oxyacids are substituted. The metal substituting the hydrogen atoms of the heteropoly acid is at least one element selected from the group consisting of Group 1, Group 2, Group 11 and Group 13 elements of the Periodic Table, and examples of onium salts of heteropoly acids include ammonium salts with ammonium or amines. Particularly preferred among these heteropoly acid salts are metal salts of lithium, sodium, potassium, cesium, magnesium, barium, copper, gold and gallium.

As heteropoly acid salts that are preferred from the standpoint of catalyst performance and practicality there may be mentioned lithium salt of tungstophosphoric acid, sodium salt of tungstophosphoric acid, copper salt of tungstophosphoric acid, lithium salt of tungstosilicic acid, sodium salt of tungstosilicic acid and copper salt of tungstosilicic acid, but there is no limitation to these.

There are also no particular restrictions on the carrier used for the catalyst of the invention (I) so long as it is a porous substance ordinarily used as a carrier. As specific preferred carriers there may be mentioned silica, diatomaceous earth, montmorillonite, titania, active carbon, alumina, silica-alumina and zeolite.

The particle size of the carrier is preferably 1 mm to 10 mm, and more preferably 3 mm to 8 mm. When the reaction is carried out by packing the catalyst into a cylindrical reactor, a particle size that is too small will result in a large pressure loss when the gas flows through, creating an inconvenience in that effective gas circulation cannot be accomplished. If the particle size is too large, the reaction gas can no longer diffuse into the catalyst interior, preventing effective functioning of the catalyst components. Regarding the fine pore structure of the carrier, it has a fine pore diameter of preferably 1 nm to 1000 nm, and more preferably 30 nm to 100 nm.

There are also no particular restrictions on the form of the carrier, and for example powders, spheres, pellets or any other desired form may be used.

The composition of (a), (b) and (c) in the catalyst according to the invention (I) containing (a) metallic palladium, (b) metallic gold and (c) at least one compound selected from among heteropoly acids and salts thereof is preferably (a) 0.1 wt% - 10.0 wt%, (b) an amount with respect to the (a) metallic palladium such that the weight ratio of (a) metallic palladium:(b) metallic gold = 1:0.1-5.0 and (c) 0.1 wt% - 90 wt%; more favorable results are obtained with (a) 0.5 wt% - 5.0 wt%, (b) an amount with respect to the (a) metallic palladium such that the weight ratio of (a) metallic palladium:(b) metallic gold = 1:0.1-5.0 and (c) 1.0 wt% - 50 wt%.

As regards the catalysts for production of acetic acid disclosed in Japanese Unexamined Patent Publication No. 7-89896 and Japanese Unexamined Patent Publication No. 9-67298 already cited above as prior art, Group 11 elements including gold are indicated as constituent elements of the catalysts under the claims and the Detailed Description of the Invention, but they nowhere mention the effectiveness of a catalyst containing metallic gold and metallic palladium in a specific proportion. The present inventors have conducted much research in this connection.

As a result, it was surprisingly found that the space time yields and selectivity for acetic acid drastically increase when the constituent elements of the catalyst for production of acetic acid according to the invention are present in amounts such that the weight ratio of (a) metallic palladium and (b) metallic gold is (a) metallic palladium:(b) metallic gold = 1:0.1-5.0, and more preferably (a) metallic palladium:(b) metallic gold = 1:0.2-1.0.

The cause of the improved catalytic activity when the weight ratio of (a) metallic palladium and (b) metallic gold is in this specified range is not yet fully understood at the present time, but it is believed to be as follows. First, metallic gold itself is a very stable substance, and metallic gold alone is not thought to exhibit activity for acetic acid synthesis by direct oxidation of ethylene. In fact, to our knowledge there is no prior literature describing direct oxidation of ethylene with metallic gold alone. Thus, the alloying of metallic gold and metallic palladium, or their interaction, may be considered to be an important factor for synthesis of acetic acid by direct oxidation of ethylene.

As a result, in small amounts wherein the weight ratio of (b) metallic gold to (a) metallic palladium is, for example, such that (a) metallic palladium:(b) metallic gold = 1:<0.1, it is thought that the metallic gold is too sparse with respect to the metallic palladium so that sufficient interaction does not occur between the metallic gold and metallic palladium and no effect is exhibited. On the other hand, in large amounts wherein (a) metallic palladium:(b) metallic gold = 1:≥5.0, it is thought that the essentially inactive metallic gold becomes too abundant, inhibiting oxidation reaction on the metallic palladium such that no effect is exhibited.

These are the reasons believed to be responsible for the optimum range for the weight ratio of (a) metallic palladium and (b) metallic gold.

The amount of metal in the catalyst according to the invention (I) can be measured in the following manner. After thoroughly pulverizing a prescribed amount of the catalyst with a mortar or the like into a uniform powder, the catalyst powder is added to an acid such as hydrofluoric acid and dissolved to prepare a homogeneous solution. The solution is then diluted to an appropriate concentration with purified water containing no ions to prepare a solution for analysis. Such solutions are quantitatively analyzed with an apparatus for inductively coupled plasma atomic emission spectroscopy (for example, an SPS-1700 manufactured by Seiko Instruments Inc.). The precision of the apparatus can be easily adjusted with commercially available standard reagents of different elements, and repeatable quantitation is possible.

The catalyst for production of acetic acid according to the invention (II) is a catalyst for production of acetic acid according to the invention (I) wherein (d) at least one element selected from the group consisting of Group 6 elements, Group 7 elements, Group 8 elements, Group 9 elements, Group 10 elements and Group 12 elements of the Periodic Table is also loaded on the carrier in addition to the (a) metallic palladium, (b) metallic gold and (c) at least one compound selected from among heteropoly acids and/or salts thereof.

The catalyst of the invention (II) is a quaternary catalyst wherein a catalyst according to the invention (I) further contains an element belonging to group (d). The (a) metallic palladium, (b) metallic gold and (c) at least one compound selected from among heteropoly acids and/or salts thereof used in the catalyst according to the invention (II) are the same as in the catalyst of the invention (I). The carrier is also the same as in the catalyst of the invention (I).

The (d) at least one element selected from the group consisting of Group 6 elements, Group 7 elements, Group 8 elements, Group 9 elements, Group 10 elements and Group 12 elements of the Periodic Table used in the catalyst of the invention (II) refers specifically to elements such as chromium, manganese, rhenium, ruthenium, rhodium, nickel and zinc. As preferred ones there may be mentioned chromium, zinc, etc.

The composition of (a), (b), (c) and (d) in the catalyst according to the invention (II) containing (a) metallic palladium, (b) metallic gold, (c) at least one compound selected from among heteropoly acids and/or salts thereof, and (d) at least one element selected from the group consisting of Group 6 elements, Group 7 elements, Group 8 elements, Group 9 elements, Group 10 elements and Group 12 elements of the Periodic Table, is preferably (a) 0.1 wt% - 10.0 wt%, (b) an amount with respect to the (a) metallic palladium such that the weight ratio of (a) metallic palladium:(b) metallic gold = 1:0.1-5.0, (c) 0.1 wt% - 90 wt% and (d) 0.01 wt% - 5.00 wt%; more favorable results are obtained with (a) 0.5 wt% - 5.0 wt%, (b) an amount with respect to the (a) metallic palladium such that the weight ratio of (a) metallic palladium:(b) metallic gold = 1:0.1-5.0, (c) 1.0 wt% - 50 wt% and (d) 0.1 wt% - 2.00 wt%.

The weight ratio of (a) metallic palladium and (b) metallic gold is important, as with the catalyst of the invention (I), and their weight ratio is preferably in the range of (a) metallic palladium:(b) metallic gold = 1:0.1-5.0, and more preferably (a) metallic palladium:(b) metallic gold = 1:0.2-1.0.

The catalyst for production of acetic acid according to the invention (III) is a catalyst for production of acetic acid according to the invention (I), wherein (e) at least one element selected from the group consisting of Group 11 elements, Group 14 elements, Group 15 elements and Group 16 elements of the Periodic Table is also loaded on the carrier in addition to the (a) metallic palladium, (b) metallic gold and (c) at least one compound selected from among heteropoly acids and/or salts thereof.

The catalyst of the invention (III) is a quaternary catalyst wherein a catalyst according to the invention (I) further contains an element belonging to group (e). The (a) metallic palladium, (b) metallic gold and (c) at least one compound selected from among heteropoly acids and/or salts thereof used in the catalyst according to the invention (III) are the same as in the catalyst of the invention (I). The carrier is also the same as in the catalyst of the invention (I).

The (e) Group 11 elements, Group 14 elements, Group 15 elements and Group 16 elements of the Periodic Table used in the catalyst of the invention (III) refers specifically to elements such as copper, silver, tin, lead, antimony, bismuth, selenium and tellurium. Tellurium is a preferred one.

The composition of (a), (b), (c) and (e) in the catalyst according to the invention (III) containing (a) metallic palladium, (b) metallic gold and (c) at least one compound selected from among heteropoly acids and/or salts thereof, with (e) at least one element selected from the group consisting of Group 11 elements, Group 14 elements, Group 15 elements and Group 16 elements of the Periodic Table, is preferably (a) 0.1 wt% - 10.0 wt%, (b) an amount with respect to the (a) metallic palladium such that the weight ratio of (a) metallic palladium:(b) metallic gold = 1:0.1-5.0, (c) 0.1 wt% - 90 wt% and (e) 0.01 wt% - 5.00 wt%; more favorable results are obtained with (a) 0.5 wt% - 5.0 wt%, (b) an amount with respect to the (a) metallic palladium such that the weight ratio of (a) metallic palladium:(b) metallic gold = 1:0.1-5.0, (c) 1.0 wt% - 50 wt% and (e) 0.1 wt% - 2.00 wt%.

The weight ratio of (a) metallic palladium and (b) metallic gold is important, as with the catalyst of the invention (I), and their weight ratio is preferably in the range of (a) metallic palladium:(b) metallic gold = 1:0.1-5.0, and more preferably (a) metallic palladium:(b) metallic gold = 1:0.2-1.0.

The catalyst for production of acetic acid according to the invention (IV) is a catalyst for production of acetic acid according to the invention (I), wherein (d) at least one element selected from the group consisting of Group 6 elements, Group 7 elements, Group 8 elements, Group 9 elements, Group 10 elements and Group 12 elements of the Periodic Table and (e) at least one element selected from the group consisting of Group 11 elements, Group 14 elements, Group 15 elements and Group 16 elements of the Periodic Table are also loaded on the carrier in addition to the (a) metallic palladium, (b) metallic gold and (c) at least one compound selected from among heteropoly acids and/or salts thereof.

The catalyst of the invention (IV) is a quinary catalyst wherein a catalyst according to the invention (I) further contains elements belonging to group (d) and group (e). The (a) metallic palladium, (b) metallic gold and (c) at least one compound selected from among heteropoly acids and/or salts thereof used in the catalyst according to the invention (IV) are the same as in the catalyst of the invention (I). The (d) at least one element selected from the group consisting of Group 6 elements, Group 7 elements, Group 8 elements, Group 9 elements, Group 10 elements and Group 12 elements of the Periodic Table is the same as in the catalyst of the invention (II). The (e) at least one element selected from the group consisting of Group 11 elements, Group 14 elements, Group 15 elements and Group 16 elements of the Periodic Table is the same as in the catalyst of the invention (III). The carrier is also the same as in the catalyst of the invention (I).

The composition of (a), (b), (c), (d) and (e) in the catalyst according to the invention (IV) containing (a) metallic palladium, (b) metallic gold, (c) at least one compound selected from among heteropoly acids and/or salts thereof, (d) at least one element selected from the group consisting of Group 6 elements, Group 7 elements, Group 8 elements, Group 9 elements, Group 10 elements and Group 12 elements of the Periodic Table and (e) at least one element selected from the group consisting of Group 11 elements, Group 14 elements, Group 15 elements and Group 16 elements of the Periodic Table, is preferably (a) 0.1 wt% - 10.0 wt%, (b) an amount with respect to the (a) metallic palladium such that the weight ratio of (a) metallic palladium:(b) metallic gold = 1:0.1-5.0, (c) 0.1 wt% - 90 wt%, (d) 0.01 wt% - 5.00 wt% and (e) 0.01 wt% - 5.00 wt%; more favorable results are obtained with (a) 0.5 wt% - 5.0 wt%, (b) an amount with respect to the (a) metallic palladium such that the weight ratio of (a) metallic palladium:(b) metallic gold = 1:0.1-5.0, (c) 1.0 wt% - 50 wt%, (d) 0.1 wt% - 2.00 wt% and (e) 0.1 wt% - 2.00 wt%.

The weight ratio of (a) metallic palladium and (b) metallic gold is important, as with the catalyst of the invention (I), and their weight ratio is preferably in the range of (a) metallic palladium:(b) metallic gold = 1:0.1-5.0, and more preferably (a) metallic palladium:(b) metallic gold = 1:0.2-1.0.

The invention (V) is a process for production of a catalyst for production of acetic acid according to any one of the inventions (I) to (IV).

A catalyst for production of acetic acid according to the invention (I) can be produced by a process comprising the following steps 1 and 2.

### Step 1

A step wherein (a) metallic palladium and (b) metallic gold are loaded on a carrier to obtain a metallic palladium-loaded catalyst.

### Step 2

A step wherein (c) at least one compound selected from among heteropoly acids and/or salts thereof is loaded on the metallic palladium-loaded catalyst obtained in step 1 to obtain a catalyst for production of acetic acid.

A catalyst for production of acetic acid according to the invention (II) can be produced by a process comprising the following steps 1 and 2.

### Step 1

A step wherein (a) metallic palladium, (b) metallic gold and (d) at least one element selected from the group consisting of Group 6 elements, Group 7 elements, Group 8 elements, Group 9 elements, Group 10 elements and Group 12 elements of the Periodic Table are loaded on a carrier to obtain a metallic palladium-loaded catalyst.

### Step 2

A step wherein (c) at least one compound selected from among heteropoly acids and/or salts thereof is loaded on the metallic palladium-loaded catalyst obtained in step 1 to obtain a catalyst for production of acetic acid.

A catalyst for production of acetic acid according to the invention (III) can be produced by a process comprising the following steps 1 and 2.

### Step 1

A step wherein (a) metallic palladium, (b) metallic gold and (e) at least one element selected from the group consisting of Group 11 elements, Group 14 elements, Group 15 elements and Group 16 elements of the Periodic Table are loaded on a carrier to obtain a metallic palladium-loaded catalyst.

### Step 2

A step wherein (c) at least one compound selected from among heteropoly acids and/or salts thereof is loaded on the metallic palladium-loaded catalyst obtained in step 1 to obtain a catalyst for production of acetic acid.

A catalyst for production of acetic acid according to the invention (IV) can be produced by a process comprising the following steps 1 and 2.

### Step 1

A step wherein (a) metallic palladium, (b) metallic gold, (d) at least one element selected from the group consisting of Group 6 elements, Group 7 elements, Group 8 elements, Group 9 elements, Group 10 elements and Group 12 elements of the Periodic Table and (e) at least one element selected from the group consisting of Group 11 elements, Group 14 elements, Group 15 elements and Group 16 elements of the Periodic Table are loaded on a carrier to obtain a metallic palladium-loaded catalyst.

### Step 2

A step wherein (c) at least one compound selected from among heteropoly acids and/or salts thereof is loaded on the metallic palladium-loaded catalyst obtained in step 1 to obtain a catalyst for production of acetic acid.

Step 1 for production of a catalyst according to the invention (I) will be explained first.

Step 1 for production of a catalyst according to the invention (I) is a step wherein (a) metallic palladium and (b) metallic gold are loaded on a carrier to obtain a metallic palladium-loaded catalyst.

There are no particular restrictions on the palladium starting material used for production of the catalyst of the invention (I). Metallic palladium may of course be used, and palladium compounds that can be converted to metallic palladium may also be used. As examples of palladium compounds that can be converted to metallic palladium there may be mentioned halides such as palladium chloride, organic acid salts such as palladium acetate, and also palladium nitrate, palladium oxide, palladium sulfate and sodium tetrachloropalladate, but there is no restriction to these.

There are also no particular restrictions on the gold starting material used for production of the catalyst of the invention (I). Metallic gold may of course be used, and gold compounds that can be converted to metallic gold may also be used. As examples of gold compounds that can be converted to metallic gold there may be mentioned halides such as auric chloride, organic salts such as gold cyanide, gold chlorotriphenylphosphine, and also gold oxide, gold sulfate and sodium tetrachloroaurate, but there is no restriction to these.

There are no particular restrictions on the method of loading the metallic palladium, or palladium compound that can be converted to metallic palladium, on the carrier. It may be accomplished by any method and, for example, when loading a palladium compound that can be converted to metallic palladium, it may be loaded on the carrier by a method whereby the palladium compound is dissolved in an appropriate solvent such as water or acetone, an inorganic or organic acid such as hydrochloric acid, nitric acid or acetic acid, or a solution thereof, after which the carrier is impregnated therewith and dried, etc.

There are no particular restrictions on the method of loading the metallic gold, or gold compound that can be converted to metallic gold, on the carrier. It may be accomplished by any method and, for example, when loading a gold compound that can be converted to metallic gold, it may be loaded on the carrier by a method whereby the gold compound is dissolved in an appropriate solvent such as water or acetone, an inorganic or organic acid such as hydrochloric acid, nitric acid or acetic acid, or a solution thereof, after which the carrier is impregnated therewith and dried, etc.

There are no particular restrictions on the method whereby a palladium compound that can be converted to metallic palladium is converted to metallic palladium after it has been loaded, and any publicly known method may be used. Specifically there may be mentioned a method whereby the palladium compound in the palladium compound-loaded catalyst is reduced to metallic palladium with a suitable reducing agent such as hydrazine or hydrogen, either directly or after treatment with sodium hydroxide or sodium metasilicate for conversion to an oxide or hydroxide.

There are also no particular restrictions on the method whereby a gold compound that can be converted to metallic gold is converted to metallic gold after it has been loaded, and any publicly known method may be used. Specifically there may be mentioned a method whereby the gold in the gold compound-loaded catalyst is reduced to metallic gold with a suitable reducing agent such as hydrazine or hydrogen, either directly or after treatment with sodium hydroxide or sodium metasilicate for conversion to an oxide or hydroxide.

The conversion procedure for a palladium compound that can be converted to metallic palladium or a gold compound that can be converted to metallic gold may be carried out after isolating the catalyst with the loaded palladium compound and gold compound, or directly following the loading procedure. If conditions permit, it is preferred for it to be carried out directly following the loading procedure, without isolation.

Step 1 for production of a catalyst according to the invention (II) will now be explained.

Step 1 for production of a catalyst according to the invention (II) is a step wherein (a) metallic palladium, (b) metallic gold and (d) at least one element selected from the group consisting of Group 6 elements, Group 7 elements, Group 8 elements, Group 9 elements, Group 10 elements and Group 12 elements of the Periodic Table are loaded on a carrier to obtain a metallic palladium-loaded catalyst.

The starting materials for the metallic palladium and metallic gold used for production of the catalyst of the invention (II), the method for their loading and the method for conversion to metallic palladium and metallic gold are the same as in the production process for the catalyst of the invention (I).

As examples of compounds including (d) at least one element selected from the group consisting of Group 6 elements, Group 7 elements, Group 8 elements, Group 9 elements, Group 10 elements and Group 12 elements of the Periodic Table used for production of a catalyst according to the invention (II) (hereunder referred to as the "first additive") there may be mentioned the elements themselves, or halides, organic acid salts, inorganic acid salts, oxides, etc. containing those elements. Specifically there may be mentioned chromium chloride, manganese chloride, rhenium chloride, ruthenium chloride, rhodium chloride, nickel chloride, zinc chloride, chromium acetate, nickel acetate, zinc acetate, chromium nitrate, zinc nitrate, chromium sulfate, zinc sulfate, chromium oxide, nickel oxide and zinc oxide, but there is no limitation to these.

There are no particular restrictions on the method of loading the first additive on the carrier. It may be accomplished by any method and, for example, when loading a halide of the element, it may be loaded on the carrier by a method whereby the halide is dissolved in an appropriate solvent such as water or acetone, an inorganic or organic acid such as hydrochloric acid, nitric acid or acetic acid, or a solution thereof, after which the carrier is impregnated therewith and dried, etc.

The loading of the first additive on the carrier, and the loading of the metallic palladium or palladium compound that can be converted to metallic palladium and metallic gold or gold compound that can be converted to metallic gold on the carrier, may be accomplished in any order. That is, the loadings may be accomplished simultaneously, or before and after each other. When the palladium starting material and the gold starting material are palladium and gold compounds that can be converted to metallic palladium and metallic gold, the loading may be accomplished after the procedure of conversion to metallic palladium and metallic gold. It is generally preferred for the loading of the first additive on the carrier to be accomplished simultaneously with loading of the metallic palladium and metallic gold, or the palladium compound and gold compound that can be converted to metallic palladium and metallic gold, on the carrier.

Step 1 for production of a catalyst according to the invention (III) will now be explained.

Step 1 for production of a catalyst according to the invention (III) is a step wherein (a) metallic palladium, (b) metallic gold and (e) at least one element selected from the group consisting of Group 11 elements, Group 14 elements, Group 15 elements and Group 16 elements of the Periodic Table are loaded on a carrier to obtain a metallic palladium-loaded catalyst.

The starting materials for the metallic palladium and metallic gold used for production of the catalyst of the invention (III), the method for their loading and the method for conversion to metallic palladium and metallic gold are the same as in the production process for the catalyst of the invention (I).

As examples of compounds including (e) at least one element selected from the group consisting of Group 11 elements, Group 14 elements, Group 15 elements and Group 16 elements of the Periodic Table used for production of a catalyst according to the invention (III) (hereunder referred to as the "second additive") there may be mentioned the elements themselves, or halides, organic acid salts, inorganic acid salts, oxides, etc. containing those elements. Specifically there may be mentioned copper chloride, silver chloride, tin chloride, lead chloride, antimony chloride, bismuth chloride, tellurium chloride, copper acetate, tin acetate, lead acetate, copper nitrate, silver nitrate, lead nitrate, copper sulfate, lead sulfate, copper oxide, telluric acid, tellurous acid, etc., but there is no limitation to these.

There are no particular restrictions on the method of loading the second additive on the carrier. It may be accomplished by any method and, for example, when loading a halide of the element, it may be loaded on the carrier by a method whereby the halide is dissolved in an appropriate solvent such as water or acetone, an inorganic or organic acid such as hydrochloric acid, nitric acid or acetic acid, or a solution thereof, after which the carrier is impregnated therewith and dried, etc.

The loading of the second additive on the carrier, and the loading of the metallic palladium and metallic gold or the palladium compound and gold compound that can be converted to metallic palladium and metallic gold on the carrier, may be accomplished in any order. They may be accomplished separately, or two or more may be accomplished simultaneously. When the palladium starting material and gold starting material are palladium and gold compounds that can be converted to metallic palladium and metallic gold, the loading may be accomplished after the procedure of conversion to metallic palladium and metallic gold. It is generally preferred for the loading of the second additive to be accomplished simultaneously with loading of the metallic palladium and metallic gold, or the palladium compound and gold compound that can be converted to metallic palladium and metallic gold, on the carrier.

Step 1 for production of a catalyst according to the invention (IV) will now be explained.

Step 1 for production of a catalyst according to the invention (IV) is a step wherein (a) metallic palladium, (b) metallic gold, (d) at least one element'selected from the group consisting of Group 6 elements, Group 7 elements, Group 8 elements, Group 9 elements, Group 10 elements and Group 12 elements of the Periodic Table and (e) at least one element selected from the group consisting of Group 11 elements, Group 14 elements, Group 15 elements and Group 16 elements of the Periodic Table are loaded on a carrier to obtain a metallic palladium-loaded catalyst.

The starting materials for the metallic palladium and metallic gold used for production of the catalyst of the invention (IV), the method for their loading and the method for conversion to metallic palladium and metallic gold are the same as in the production process for the catalyst of the invention (I).

The specific examples for the first additive used for production of the catalyst of the invention (IV) and the method of its loading are the same as in the production process for the catalyst of the invention (II).

The specific examples for the second additive used for production of the catalyst of the invention (IV) and the method of its loading are the same as in the production process for the catalyst of the invention (III).

The loading of the metallic palladium or palladium compound that can be converted to metallic palladium, the metallic gold or gold compound that can be converted to metallic gold, the first additive and the second additive on the carrier, may be accomplished in any order. They may be accomplished separately, or two or more may be accomplished simultaneously. When the palladium starting material is a palladium compound that can be converted to metallic palladium, or when the gold starting material is a gold compound that can be converted to metallic gold, the first additive and second additive may each be loaded separately before the procedure of conversion to metals, or they may be loaded after the conversion procedure. It is generally preferred for their loading to be accomplished simultaneously with loading of the metallic palladium and metallic gold, or the palladium compound and gold compound that can be converted to metallic palladium and metallic gold, on the carrier.

The metallic palladium- and metallic gold-loaded catalyst obtained in this manner can be isolated by filtration followed by washing and drying, by conventional methods.

Step 2 is a step wherein a heteropoly acid and/or a salt thereof is loaded on the metallic palladium-loaded catalyst obtained in step 1 to obtain a catalyst for production of acetic acid.

There are no particular restrictions on heteropoly acids that can be used for production of a catalyst according to the present inventions (I) to (IV), and they may be heteropoly acids containing phosphorus, silicon, boron, aluminum, germanium, titanium, zirconium, cerium, cobalt or chromium as the hetero atom and at least one element selected from the group consisting of molybdenum, tungsten, vanadium, niobium and tantalum as the polyatom. As specifically preferred ones there may be mentioned tungstosilicic acid, tungstophosphoric acid, molybdosilicic acid, molybdophosphoric acid, molybdotungstophosphoric acid, molybdotungstosilicic acid, vanadotungstophosphoric acid, vanadotungstosilicic acid, vanadomolybdosilicic acid, tungstoboric acid, molybdoboric acid and molybdotungstoboric acid. Particularly preferred among these are heteropoly acids wherein the hetero atom is phosphorus or silicon and the polyatom is at least one element selected from the group consisting of tungsten, molybdenum and vanadium.

The salt of the heteropoly acid is a metal salt or onium salt wherein all or a portion of the hydrogen atoms of the acid produced by condensation of the two or more different inorganic oxyacids are substituted. The metal substituting the hydrogen atoms of the heteropoly acid is at least one element selected from the group consisting of Group 1, Group 2, Group 11 and Group 13 elements of the Periodic Table, and examples of onium salts of heteropoly acids include ammonium salts with ammonium or amines. Particularly preferred among these heteropoly acid salts are metal salts of lithium, sodium, potassium, cesium, magnesium, barium, copper gold and gallium.

As heteropoly acid salts that are preferred from the standpoint of catalyst performance and practicality there may be mentioned lithium salt of tungstophosphoric acid, sodium salt of tungstophosphoric acid, copper salt of tungstophosphoric acid, lithium salt of tungstosilicic acid, sodium salt of tungstosilicic acid and copper salt of tungstosilicic acid, but there is no limitation to these.

There are no particular restrictions on the method of loading the heteropoly acid or salt thereof on the carrier, and any publicly known method may be used. As examples there may be mentioned such means as the impregnation method, spray impregnation method, evaporation to dry hardness method, kneading method and adsorption method, but there is no limitation to these. The solvent used for impregnation may be any one which can dissolve the heteropoly acid, and water, organic solvents and their mixtures may be used. Water, alcohol and the like are preferred.

The invention (VI) will now be explained. The invention (VI) is a process for production of acetic acid whereby acetic acid is synthesized from ethylene and oxygen by a gas phase one-stage reaction, using a catalyst for production of acetic acid according to any one of the inventions (I) to (IV).

In the process for production of acetic acid according to the invention (VI), there is no particular restriction on the reaction temperature for reaction of the ethylene and oxygen to produce acetic acid. It is preferably 100°C-300°C, and more preferably 140°C-250°C. In terms of the equipment it is advantageous in practice for the reaction pressure to be from 0.0 MPa (gauge pressure) to 3.0 MPa (gauge pressure), but there is no particular restriction thereto. It is more preferably in the range of 0.1 MPa (gauge pressure) to 1.5 MPa (gauge pressure).

In the process for production of acetic acid according to the invention (VI), the gas supplied to the reaction system contains ethylene and oxygen, and if necessary nitrogen, carbon dioxide, a rare gas or the like may also be used as a diluent.

The ethylene is supplied to the reaction system in an amount corresponding to a proportion of 5% to 80% by volume, and preferably 10% to 50% by volume, and the oxygen in an amount corresponding-to a proportion of 1% to 15% by volume, and preferable 3% to 10% by volume, with respect to the total amount of gas supplied.

The presence of water in the reaction system will provide a notable effect of improved acetic acid production activity and selectivity, as well as prolonged activity of the catalyst in the reaction system. It is suitable for water vapor to be included in the reaction gas at 1% to 60% by volume, but it is preferably included at 5% to 40% by volume.

When carrying out the process for production of acetic acid according to the invention (VI) it is preferred to use a high purity ethylene starting material, but there is no problem with mixing a lower saturated hydrocarbon such as methane, ethane or propane. The oxygen can also be in a form such as air, diluted with an inert gas such as nitrogen, carbon dioxide gas or the like, but when the reaction gas is circulated it is generally more advantageous to use oxygen at a high concentration, preferably 99% or greater.

The reaction mixture gas is preferably passed through the catalyst at a space velocity (SV) of 10/h to 10,000/h, and especially 300/h to 5000/h, in a standard state.

The reaction system is not particularly restricted, and any publicly known process may be used employing a system such as a fixed bed, fluidized bed or the like. It is more advantageous in practical terms to employ a fixed bed having corrosion-resistant reaction tubes packed with the catalyst.

The catalyst for production of acetic acid according to the invention (VII) is an acetic acid production catalyst that is used in a process for production of acetic acid which comprises reacting ethylene and oxygen, and that contains (a) metallic palladium and (b) a heteropoly acid salt, wherein the (b) heteropoly acid salt comprises a metal salt wherein all or a portion of the hydrogen atoms of the heteropoly acid are replaced with at least one element selected from the group consisting of Group 6 elements, Group 7 elements, Group 9 elements and Group 10 elements of the Periodic Table, or the same catalyst loaded on a carrier.

The palladium contained in the catalyst according to the invention (VII) is metallic palladium. There are no particular restrictions on the heteropoly acid as the starting material for the heteropoly acid salt, and it may be a heteropoly acid containing phosphorus, silicon, boron, aluminum, germanium, titanium, zirconium, cerium, cobalt or chromium as the hetero atom and at least one element selected from the group consisting of molybdenum, tungsten, vanadium, niobium and tantalum as the polyatom.

As specific examples there may be mentioned tungstosilicic acid, tungstophosphoric acid, molybdosilicic acid, molybdophosphoric acid, molybdotungstophosphoric acid, molybdotungstosilicic acid, vanadotungstophosphoric acid, vanadotungstosilicic acid, vanadomolybdosilicic acid, tungstoboric acid, molybdoboric acid and molybdotungstoboric acid.

Particularly preferred among these are heteropoly acids wherein the hetero atom is phosphorus or silicon and the polyatom is at least one element selected from the group consisting of tungsten, molybdenum and vanadium.

The metal replacing all or a portion of the hydrogen atoms in the heteropoly acid of the catalyst for production of acetic acid according to the invention (VII) is at least one element selected from the group consisting of Group 6 elements, Group 7 elements, Group 9 elements and Group 10 elements of the Periodic Table. Among them, chromium, manganese, cobalt and nickel are particularly preferred.

As specific preferred heteropoly acid salts there may be mentioned chromium salt of tungstophosphoric acid, manganese salt of tungstophosphoric acid, cobalt salt of tungstophosphoric acid, nickel salt of tungstophosphoric acid, chromium salt of tungstosilicic acid, manganese salt of tungstosilicic acid, cobalt salt of tungstosilicic acid and nickel salt of tungstosilicic acid, but there is no limitation to these.

Although the structure of the catalyst for production of acetic acid according to the invention (VII) is not precisely known, the palladium of group (a) is not a compound such as palladium chloride, palladium oxide or a heteropoly acid palladium salt but rather metallic palladium, while the heteropoly acid salt belonging to group (b) is unlike a compound oxide, in that it is a compound exhibiting acidity with a definite structure as a metal salt wherein all or a portion of the hydrogen atoms of an inorganic poly acid are replaced. The elements and compounds of these two groups (a) and (b) are believed to exist very close together as compounds, the compounds and elements of each group interacting to express very high activity and selectivity, and are believed to exhibit excellent acetic acid-producing activity and selectivity at lower reaction temperatures than heteropoly acid palladium salt catalysts (Japanese Unexamined Patent Publication No. 54-57488) and palladium-containing ternary oxygen compound catalysts (Japanese Examined Patent Publication No. 46-6763).

The catalyst of the invention (VII) has all or a portion of the hydrogen atoms of the heteropoly acid replaced by an element belonging to a different Group than the heteropoly acid salt in the binary catalyst described in Japanese Unexamined Patent Publication No. 7-89896. Also, despite the disclosure of a system where Group 6 elements, Group 7 elements, Group 9 elements and Group 10 elements are added as auxiliary catalysts in the ternary and quaternary catalysts described in Japanese Unexamined Patent Publication No. 9-67298, these elements are merely added in the form of compounds unrelated to heteropoly acids or as alloys with metallic palladium.

The catalyst of the invention (VII) has all or a portion of the hydrogen atoms of the heteropoly acid replaced with at least one element selected from the group consisting of Group 6 elements, Group 7 elements, Group 9 elements and Group 10 elements, and thereby has the characteristic of higher activity than conventional acetic acid production catalysts comprising metallic palladium-heteropoly acid salts, as well as excellent stability with low catalyst deterioration.

The compositional ratio of (a) and (b) in the catalyst-containing the (a) metallic palladium and (b) heteropoly acid salt, as the catalyst of the invention (VII), is preferably (a) 1 gram of atoms:(b) 0.025 gram of molecules to 500 grams of molecules, and especially (a) 1 gram of atoms:(b) 0.1 gram of molecules to 400 grams of molecules, in order to achieve a more desirable result.

There are no particular restrictions on the palladium content of the catalyst of the invention (VII). It is preferably in the range of 0.01-6 wt%, and more preferably in the range of 0.1-2 wt%. Although the reaction will still proceed even if the palladium content is over 6 wt%, the high price of palladium renders this uneconomical and unpractical.

The catalyst for production of acetic acid according to the invention (VIII) is an acetic acid production catalyst that is used in a process for production of acetic acid which comprises reacting ethylene and oxygen, and that contains (a) metallic palladium, (b) a heteropoly acid salt and (c) at least one element selected from the group consisting of Group 8 elements, Group 11 elements, Group 12 elements, Group 14 elements, Group 15 elements and Group 16 elements of the Periodic Table, wherein the (b) heteropoly acid salt comprises a metal salt wherein all or a portion of the hydrogen atoms of the heteropoly acid are replaced with at least one element selected from the group consisting of Group 6 elements, Group 7 elements, Group 9 elements and Group 10 elements of the Periodic Table, or the same catalyst loaded on a carrier.

The catalyst for production of acetic acid according to the invention (VIII) is a ternary catalyst wherein a metal element belonging to group (c) has been added to a catalyst of the invention (VII).

The (a) metallic palladium and (b) heteropoly acid salt used for the invention (VIII) are the same as for the invention (VII). When loaded on a carrier, the carrier is also the same as for the invention (VII).

The group (c) element used for the invention (VIII) may be, specifically, ruthenium, copper, silver, gold, zinc, tin, lead, antimony, bismuth, selenium, tellurium or the like, but there is no limitation to these. Preferred for the group (c) element are gold, zinc, bismuth, selenium and tellurium.

Although the structure of the catalyst for production of acetic acid according to the invention (VIII) is not precisely known, the palladium of group (a) and the heteropoly acid salt belonging to group (b) are the same as for the invention (VII), and the same effect may be expected as by the invention (VII). The element belonging to group (c) is not in the form of a structural element of the heteropoly acid or an element replacing the hydrogen atoms of the heteropoly acid, but rather that of a metal, a compound or an alloy with metallic palladium. The elements and compounds of these three groups (a), (b) and (c) are believed to exist very close together. The metallic palladium and the compounds and elements of each group represented by (b) and (c) therefore interact expressing very high activity and selectivity, and are believed to exhibit excellent acetic acid-producing activity and selectivity at lower reaction temperatures than conventional catalysts.

The compositional ratio of (a), (b) and (c) in the catalyst containing the (a) metallic palladium, (b) heteropoly acid salt and group (c) element of the catalyst of the invention (VIII) is preferably (a) 1 gram of atoms:(b) 0.025 gram of molecules to 500 grams of molecules:(c) 0.005 gram of atoms to 10 grams of atoms, and especially (a) 1 gram of atoms:(b) 0.1 gram of molecules to 400 grams of molecules:(c) 0.01 gram of atoms to 5 grams of atoms, in order to achieve a more desirable result.

There are no particular restrictions on the content of the group (c) element, but it is preferably in the range of 0.01-30 wt% with respect to the total of the catalyst components.

The catalyst for production of acetic acid according to the invention (VII) or (VIII) may be effectively used with only the catalyst substance having the aforementioned composition, or it may be loaded on a carrier for more convenient use.

There are no particular restrictions on the carrier used for loading of the catalyst of the invention (VII) or (VIII) so long as it is a porous substance ordinarily used as a carrier. As specific carriers there may be mentioned silica, diatomaceous earth, montmorillonite, titania, active carbon, alumina and silica-alumina, which may be in the form of powders, spheres, pellets or any other desired form.

There are no particular restrictions on the particle size of the carrier. It is preferably 1 mm to 10 mm, and more preferably 3 mm to 8 mm. When the reaction is carried out by packing the catalyst into a cylindrical reactor, a particle size that is too small will result in a large pressure loss when the gas flows through, and this may result in problems such as an inability to achieve effective gas circulation. If the particle size is too large, the reaction gas can no longer diffuse into the catalyst interior, and this may prevent effective functioning of the catalyst component.

Regarding the fine pore structure of the carrier, it has a fine pore diameter of preferably 1 nm to 1000 nm, and more preferably 30 nm to 100 nm.

There are no particular restrictions on the loading amount of the heteropoly acid salt when the catalyst is loaded onto a carrier, and it will differ depending on the particle size and fine pore structure of the carrier. It is preferably in the range of 5-200 wt%, and more preferably in the range of 10-100 wt% with respect to the carrier.

The amount of metal in the catalyst according to the invention (VII) or (VIII) can be measured in the following manner. After thoroughly pulverizing a given measured amount of the catalyst with a mortar or the like into a uniform powder, the catalyst powder is heated and dissolved in an acid such as hydrofluoric acid to prepare a homogeneous solution. If necessary the solution is then diluted to an appropriate concentration with purified water containing no ions, and quantitatively analyzed with an apparatus for inductively coupled plasma atomic emission spectroscopy (for example, an SPS-1700 manufactured by Seiko Instruments Inc.). The precision of the apparatus can be easily adjusted with commercially available standard reagents of different elements, and repeatable quantitation is possible.

The invention (IX) is a process for production of a catalyst for production of acetic acid according to either the invention (VII) or (VIII).

A catalyst for production of acetic acid according to the invention (VII) can be produced by a process comprising the following steps 1 and 2.

### Step 1

A step wherein a suspension of (a) metallic palladium is obtained.

### Step 2

A step wherein the (b) heteropoly acid salt is dissolved or suspended in the metallic palladium suspension obtained in step 1, and the solvent is removed to obtain a catalyst for production of acetic acid.

For step 1, metallic palladium (palladium carbon, etc.) is used as the palladium starting material and the metallic palladium may be simply suspended in a solvent, but it may also be obtained by dissolving or suspending a palladium compound in a solvent by a well-known method to reduce it.

There are no particular restrictions on the palladium compound for the metallic palladium starting material, and compounds that can be converted to metallic palladium and may be generally used include halides such as palladium chloride, organic acid salts such as palladium acetate, and also palladium nitrate, palladium oxide, palladium sulfate and sodium tetrachloropalladate.

The solvent used may be either inorganic or organic, such as water or acetone, but there is no restriction to these so long as stirring in the solvent allows uniform dissolution or suspension of the metallic palladium or palladium compound that can be converted to metallic palladium.

When a palladium compound that can be converted to metallic palladium is used, there are no particular restrictions on the method whereby the palladium compound is afterwards converted to metallic palladium, and any publicly known method may be used. Specifically there may be mentioned a method whereby the palladium compound in the solution in which the palladium compound has been dissolved or suspended is reduced to metallic palladium with a suitable reducing agent such as hydrazine or hydrogen.

A metallic palladium suspension may be obtained in this manner.

Step 2 is a step wherein the (b) heteropoly acid salt is dissolved or suspended in the metallic palladium suspension obtained in step 1, and the solvent is removed to obtain a catalyst for production of acetic acid.

The heteropoly acid used in this step is the same as for the invention (VII).

The method of dissolving or suspending the heteropoly acid salt is not particularly restricted and may be any one that gives a homogeneous solution by stirring, etc. For example, if it will dissolve it may be dissolved directly in the metallic palladium suspension, and even if it does not dissolve, a method may be employed whereby the heteropoly acid salt is dissolved in an appropriate solvent and the solution dropped into the metallic palladium suspension.

The method of removing the solvent is not particularly restricted and may be any well-known method such as heating, pressure reduction, etc. In the case of heating, the temperature is preferably not above about 350°C as this will tend to destroy the skeleton of the heteropoly acid salt, thus impairing the acetic acid producing-activity and selectivity.

The catalyst for production of acetic acid according to the invention (VIII) can be produced by adding (c) at least one element selected from the group consisting of Group 8 elements, Group 11 elements, Group 12 elements, Group 14 elements, Group 15 elements and Group 16 elements of the Periodic Table to either or both the (a) metallic palladium in step 1 and the (b) heteropoly acid salt in step 2 as explained above for production of the catalyst of the invention (VII).

Step 1 will now be explained. The (a) metallic palladium used in step 1 is the same as described for step 1 for production of the catalyst of the invention (VII).

For addition of the elements of group (c) in step 1, there are no particular restrictions on the starting compounds for those elements (ruthenium, copper, silver, gold, zinc, tin, lead, antimony, bismuth, selenium, tellurium, etc.), and typical examples include halides such as copper chloride, zinc chloride, bismuth chloride, antimony chloride, selenium chloride and tellurium chloride; oxides such as copper oxide, zinc oxide, bismuth oxide, antimony oxide, selenium oxide and tellurium oxide; nitrates such as copper nitrate, silver nitrate, zinc nitrate and lead nitrate; acetates such as copper acetate, zinc acetate, tin acetate and lead acetate; as well as telluric acid, tellurous acid, sodium tellurite, selenic acid, selenous acid, potassium selenite, potassium antimonate, antimony sulfide, bismuth sulfide, copper sulfate, etc. Any of the metals may be used as desired.

The-method of dissolving or suspending the starting compound for the element of group (c) is not particularly restricted and may be any one that gives a homogeneous solution by stirring, etc. with the palladium starting material. For example, if it will dissolve it may be dissolved directly with the palladium starting material, and even if it does not dissolve, a method may be employed whereby the starting compound for the element of group (c) is dissolved in an appropriate solvent and the solution dropped into a solution in which the palladium starting material has been dissolved or suspended.

There are no particular restrictions on the method whereby the palladium compound that can be converted to metallic palladium and/or the starting compound for the group (c) element are converted to their respective metal elements after being loaded on the carrier, and any publicly known method may be used. Specifically there may be mentioned a method of reduction to metal with a suitable reducing agent such as hydrazine or hydrogen.

A metallic palladium suspension containing a group (c) element may be obtained in this manner.

The (b) heteropoly acid salt used in step 2 is the same as for the invention (VII), and the method of dissolving or suspending the heteropoly acid salt and the method of removing the solvent are the same as in step 2 for production of the catalyst of the invention (VII).

For addition of the elements of group (c) in step 2, the starting compounds for those elements are not subjected to a reduction procedure after being loaded and must therefore be compounds that can be easily reduced to metal elements by the metallic palladium, etc. loaded in step 1. As examples there may be mentioned telluric acid, tellurous acid, sodium tellurite, selenic acid, selenous acid, potassium selenite, potassium antimonate, and the like. Any of the metals may be used as desired.

The method of dissolving or suspending the starting compound for the element of group (c) is not particularly restricted and may be any one that gives a homogeneous solution by stirring, etc. with the heteropoly acid salt. For example, if it will dissolve it may be dissolved directly with the heteropoly acid salt, and even if it does not dissolve, a method may be employed whereby the starting compound for the element of group (c) is dissolved in an appropriate solvent and the solution dropped into a solution in which the heteropoly acid salt has been dissolved or suspended.

The invention (X) is a process for production of a carrier-loaded catalyst for production of acetic acid according to the invention (VII) or (VIII).

A carrier-loaded catalyst for production of acetic acid according to the invention (VII) can be produced by a process comprising the following steps 1 and 2.

### Step 1

A step wherein metallic palladium is loaded to obtain a metallic palladium-loaded catalyst.

### Step 2

A step wherein a heteropoly acid salt is loaded on the metallic palladium-loaded catalyst obtained in step 1 to obtain a catalyst for production of acetic acid.

There are no particular restrictions on the starting material for the metallic palladium used in step 1, and generally there may be used compounds that can be converted to metallic palladium, for example, halides such as palladium chloride, organic acid salts such as palladium acetate, and also palladium nitrate, palladium oxide, palladium sulfate and sodium tetrachloropalladate, as well as metallic palladium itself.

There are no particular restrictions on the method of loading the metallic palladium or the palladium compound that can be converted to metallic palladium on the carrier, and any method may be employed. For example, when loading a palladium compound that can be converted to metallic palladium, the palladium compound may be dissolved or suspended in an appropriate solvent such as water or acetone, in an inorganic acid or organic acid such as hydrochloric acid, nitric acid, acetic acid, or the like, or a solution thereof, and then impregnated into the carrier and dried, as the method of loading onto the carrier.

When a palladium compound that can be converted to metallic palladium is loaded on the carrier, there are no particular restrictions on the method of subsequently converting the palladium compound to metallic palladium, and any publicly known method may be used. Specifically there may be mentioned a method whereby the palladium compound-loaded catalyst is treated either directly or in an aqueous solution of sodium hydroxide, sodium metasilicate, or the like for conversion to palladium oxide or hydroxide, and is then reduced to metallic palladium with a suitable reducing agent such as hydrazine or hydrogen.

If desired, the remaining alkali salt of sodium, etc. may then be filtered by a common method, washed and dried.

A metallic palladium-loaded catalyst may be obtained in this manner.

Step 2 of this process is a step wherein the heteropoly acid salt is loaded on the metallic palladium-loaded catalyst obtained in step 1, to obtain a catalyst for production of acetic acid.

The heteropoly acid salt used in this step is the same as for the invention (VII) described above.

There are no particular restrictions on the method of loading the heteropoly acid salt on the carrier, and any publicly known method may be employed. As examples there may be mentioned such means as the impregnation method, the evaporation to dry hardness method, the kneading method and the adsorption method, but there is no limitation to these. The solvent used for impregnation may be any one which can dissolve the heteropoly acid, and water, organic solvents and their mixtures may be used. Water, alcohol, carboxylic acids and the like are preferred. However, it is not preferred for the heating temperature for drying and reduction after loading to be higher than about 350°C, as this will tend to destroy the skeleton of the heteropoly acid salt, thus impairing the acetic acid producing-activity and selectivity.

The carrier-loaded catalyst for production of acetic acid according to the invention (VIII) can be produced by adding (c) at least one element selected from the group consisting of Group 8 elements, Group 11 elements, Group 12 elements, Group 14 elements, Group 15 elements and Group 16 elements of the Periodic Table to either or both the (a) metallic palladium in step 1 and the (b) heteropoly acid salt in step 2 for production of the carrier-loaded catalyst of the invention (VII).

The metallic palladium used in step I is the same as in step 1 for production of the carrier-loaded catalyst of the invention (VII), according to the invention (X).

For addition of the elements of group (c) in step 1, the starting compounds for those elements may be the same as mentioned for step 1 for production of the catalyst of the invention (VIII), according to the invention (IX).

There are no particular restrictions on the method of loading the group (c) element or its starting compound on the carrier, and any method may be employed. For example, when loading a halide of the element, the halide may be dissolved or suspended in an appropriate solvent such as water or acetone, in an inorganic acid or organic acid such as hydrochloric acid, nitric acid, acetic acid, or the like, or a solution thereof, and then impregnated into the carrier and dried, as the method of loading onto the carrier.

The loading of the group (c) element or its starting compound on the carrier and the loading of the metallic palladium or palladium compound that can be converted to metallic palladium on the carrier may be carried out in any order. That is, both loadings may be carried out simultaneously, or one before the other. Preferred and most common is simultaneous loading on the carrier of the group (c) element or its starting compound and the metallic palladium or palladium compound that can be converted to metallic palladium.

When a palladium compound that can be converted to metallic palladium and/or a starting material for a group (c) element are loaded on the carrier, there are no particular restrictions on the method of subsequently converting the compounds to their respective metal elements, and any publicly known method may be used. Specifically there may be mentioned a method whereby the palladium compound-loaded catalyst is treated either directly or in an aqueous solution of sodium hydroxide, sodium metasilicate or the like for conversion to palladium oxide or hydroxide, and is then reduced to metallic palladium with a suitable reducing agent such as hydrazine or hydrogen.

If desired, the remaining alkali salt of sodium, etc. may then be filtered by a common method, washed and dried.

A metallic palladium-loaded catalyst may be obtained in this manner.

The heteropoly acid salt used in step 2 is the same as for the invention (VII) mentioned above, and the method of loading on the carrier is the same as described for step 2 for production of the carrier-loaded catalyst of the invention (VII), according to the invention (X).

When the group (c) element is added in step 2, the starting compound for the element is the same as described for step 2 for production of the catalyst of the invention (VIII), according to the invention (IX).

The method of loading the group (c) element or its starting compound on the carrier is the same as in step 1 for production of the carrier-loaded catalyst of the invention (VIII), according to the invention (X).

The loading of the group (c) element or its starting compound on the carrier and the loading of the heteropoly acid salt on the carrier may be carried out in any order. That is, both loadings may be carried out simultaneously, or one before the other.

The invention (XI) is a process whereby acetic acid is synthesized from ethylene and oxygen by a gas phase one-stage reaction, using a catalyst for production of acetic acid according to the invention (VII) or (VIII).

In the process for production of acetic acid according to the invention (XI), there is no particular restriction on the reaction temperature for reaction of the ethylene and oxygen to produce acetic acid, but it is preferably 100°C-300°C, and more preferably 140°C-250°C. In terms of the equipment it is advantageous in practice for the reaction pressure to be from 0 MPa (gauge pressure) to 3 MPa (gauge pressure), but it is more preferably in the range of 0.1 MPa (gauge pressure) to 1.5 MPa (gauge pressure).

The gas supplied to the reaction system according to the invention contains ethylene and oxygen, and if necessary nitrogen, carbon dioxide, a rare gas or the like may also be used as a diluent.

The ethylene is supplied to the reaction system in an amount corresponding to a proportion of 2% to 80% by volume, and preferably 5% to 50% by volume, and the oxygen in an amount corresponding to a proportion of 1% to 15% by volume, and preferably 3% to 10% by volume, with respect to the total amount of gas supplied.

The presence of water in the reaction system will provide a notable effect of improved acetic acid production activity and selectivity, as well as prolonged activity of the catalyst.

It is suitable for water vapor to be included in the reaction gas at 1% to 50% by volume, but it is preferably included at 5% to 30% by volume.

When carrying out the process of the invention it is advantageous to use a high purity ethylene starting material, but there is no problem with mixing a small amount of a lower saturated hydrocarbon such as methane, ethane or propane. The oxygen can also be in a form such as air, diluted with an inert gas such as nitrogen, carbon dioxide gas, etc., but when the reaction gas is circulated it is generally more advantageous to use oxygen at a high concentration, preferably 99% or greater.

The reaction mixture gas is preferably passed through the catalyst at a space velocity (SV) of 10 Hr⁻¹ to 10,000 Hr⁻¹, and especially 300 Hr⁻¹ to 5000 Hr⁻¹, in a standard state.

The reaction system is not particularly restricted, and any publicly known process may be used employing a system such as a fixed bed, fluidized bed or the like; however, it is more advantageous in practical terms to employ a fixed bed having corrosion-resistant reaction tubes packed with the catalyst.

The present invention will now be explained in greater detail by way of the following examples, which are only generally illustrative of the invention and are not intended to restrict it in any way.

### Example 1 (Production of acetic acid production catalyst 1)

After measuring out 2.81 g of sodium tetrachloropalladate and 1.05 g of auric chloride, purified water was added thereto, for dissolution, to 45 ml to prepare aqueous solution (1). A 69.6 g portion of a silica carrier (KA-1 carrier, 5 mmφ, product of Züd-chemie AG) was added to the beaker in which aqueous solution (1) had been prepared, for complete absorption of aqueous solution (1) onto the silica carrier.

After measuring out 8.00 g of sodium metasilicate into a separate beaker, 100 g of purified water was added thereto, for dissolution, to prepare aqueous solution (2). The silica carrier which had absorbed aqueous solution (1) was added to the beaker in which aqueous solution (2) had been prepared, and the mixture was allowed to stand at room temperature for 20 hours. Next, 8.00 g of hydrazine monohydrate was slowly added thereto while stirring at room temperature. The mixture was stirred for 4 hours after adding the hydrazine monohydrate. After filtering the catalyst and then passing purified water through for 40 hours for washing, it was dried for 4 hours under an air stream at 110°C.

In a separate beaker there was measured out 23.98 g of tungstosilicic acid 26 hydrate, and purified water was added thereto, for dissolution, to 45 ml to prepare aqueous solution (3). A metallic palladium-loaded catalyst washed with an acidic solution was added to the beaker in which aqueous solution (3) had been prepared, for complete absorption of aqueous solution (3). This was then dried for 4 hours under an air stream at 110°C to obtain acetic acid production catalyst 1.

### Example 2 (Production of acetic acid production catalyst 2)

Acetic acid production catalyst 2 was obtained by the same production process as Example 1, except that in the preparation of aqueous solution (3) in Example 1, 23.98 g of tungstophosphoric acid 26 hydrate was used instead of the 23.98 g of tungstosilicic acid 26 hydrate.

### Example 3 (Production of acetic acid production catalyst 3)

Acetic acid production catalyst 3 was obtained by the same production process as Example 1, except that in the preparation of aqueous solution (3) in Example 1, 23.98 g of tungstosilicic acid 26 hydrate was measured out, purified water was added, for dissolution, to 30 ml, and then a solution prepared by measuring out 0.0494 g of lithium nitrate and adding purified water thereto, for dissolution, to 15 ml was slowly added dropwise to the previous solution while stirring to prepare aqueous solution (3).

### Example 4 (Production of acetic acid production catalyst 4)

After measuring out 2.81 g of sodium tetrachloropalladate, 1.05 g of auric chloride and 0.1402 g of zinc chloride, purified water was added thereto, for dissolution, to 45 ml to prepare aqueous solution (1). A 69.6 g portion of a silica carrier (KA-1 carrier, 5 mmφ, product of Züd-chemie AG) was added to the beaker in which aqueous solution (1) had been prepared, for complete absorption of aqueous solution (1) onto the silica carrier.

After measuring out 8.00 g of sodium metasilicate into a separate beaker, 100 g of purified water was added thereto, for dissolution, to prepare aqueous solution (2). The silica carrier which had absorbed aqueous solution (1) was added to the beaker in which aqueous solution (2) had been prepared, and the mixture was allowed to stand at room temperature for 20 hours. Next, 8.00 g of hydrazine monohydrate was slowly added thereto while stirring at room temperature. The mixture was stirred for 4 hours after adding the hydrazine monohydrate. After filtering the catalyst and then passing purified water through for 40 hours for washing, it was dried for 4 hours under an air stream at 110°C.

In a separate beaker there was measured out 23.98 g of tungstosilicic acid 26 hydrate, and purified water was added thereto, for dissolution, to 45 ml to prepare aqueous solution (3). -A metallic palladium-loaded catalyst washed with an acidic solution was added to the beaker in which aqueous solution (3) had been prepared, for complete absorption of aqueous solution (3). This was then dried for 4 hours under an air stream at 110°C to obtain acetic acid production catalyst 4.

### Example 5 (Production of acetic acid production catalyst 5)

Acetic acid production catalyst 5 was obtained by the same production process as Example 4, except that in the preparation of aqueous solution (1) in Example 4, 0.1629 g of chromium chloride was used instead of 0.1402 g of zinc chloride.

### Example 6 (Production of acetic acid production catalyst 6)

After measuring out 2.81 g of sodium tetrachloropalladate and 1.05 g of auric chloride, purified water was added thereto, for dissolution, to 45 ml to prepare aqueous solution (1). A 69.6 g portion of a silica carrier (KA-1 carrier, 5 mmφ, product of Züd-chemie AG) was added to the beaker in which aqueous solution (1) had been prepared, for complete absorption of aqueous solution (1) onto the silica carrier.

After measuring out 8.00 g of sodium metasilicate into a separate beaker, 100 g of purified water was added thereto, for dissolution, to prepare aqueous solution (2). The silica carrier which had absorbed aqueous solution (1) was added to the beaker in which aqueous solution (2) had been prepared, and the mixture was allowed to stand at room temperature for 20 hours. Next, 8.00 g of hydrazine monohydrate was slowly added thereto while stirring at room temperature. The mixture was stirred for 4 hours after adding the hydrazine monohydrate. After filtering the catalyst and then passing purified water through for 40 hours for washing, it was dried for 4 hours under an air stream at 110°C.

Next, 0.266 g of sodium tellurite was measured out, and 45 g of purified water was added thereto to prepare aqueous solution (3). The metallic palladium-loaded carrier was added to aqueous solution (3) for complete absorption of aqueous solution (3). It was then dried for 4 hours under an air stream at 110°C to obtain a tellurium-added metallic palladium-loaded catalyst.

In a separate beaker there was measured out 23.98 g of tungstosilicic acid 26 hydrate, and purified water was added thereto, for dissolution, to 45 ml to prepare aqueous solution (4). A metallic palladium-loaded catalyst washed with an acidic solution was added to the beaker in which aqueous solution (4) had been prepared, for complete absorption of aqueous solution (4). This was then dried for 4 hours under an air stream at 110°C to obtain acetic acid production catalyst 6.

### Example 7 (Production of acetic acid production catalyst 7)

Acetic acid production catalyst 7 was obtained by the same production process as Example 6, except that in the preparation of aqueous solution (4) in Example 6, an aqueous acetic acid solution containing 0.5201 g of bismuth chloride was used instead of 0.266 g of sodium tellurite.

### Example 8 (Production of acetic acid production catalyst 8)

After measuring out 2.81 g of sodium tetrachloropalladate, 1.05 g of auric chloride and 0.1402 g of zinc chloride, purified water was added thereto, for dissolution, to 45 ml to prepare aqueous solution (1). A 69.6 g portion of a silica carrier (KA-1 carrier, 5 mmφ, product of Züd-chemie AG) was added to the beaker in which aqueous solution (1) had been prepared, for complete absorption of aqueous solution (1) onto the silica carrier.

After measuring out 8.00 g of sodium metasilicate into a separate beaker, 100 g of purified water was added thereto, for dissolution, to prepare aqueous solution (2). The silica carrier which had absorbed aqueous solution (1) was added to the beaker in which aqueous solution (2) had been prepared, and the mixture was allowed to stand at room temperature for 20 hours. Next, 8.00 g of hydrazine monohydrate was slowly added thereto while stirring at room temperature. The mixture was stirred for 4 hours after adding the hydrazine monohydrate. After filtering the catalyst and then passing purified water through for 40 hours for washing, it was dried for 4 hours under an air stream at 110°C.

Next, 0.266 g of sodium tellurite was measured out, and 45 g of purified water was added thereto to prepare aqueous solution (3). The metallic palladium-loaded carrier was added to aqueous solution (3) for complete absorption of aqueous solution (3). It was then dried for 4 hours under an air stream at 110°C to obtain a tellurium-added metallic palladium-loaded catalyst.

In a separate beaker there was measured out 23.98 g of tungstosilicic acid 26 hydrate, and purified water was added thereto, for dissolution, to 45 ml to prepare aqueous solution (4). A metallic palladium-loaded catalyst washed with an acidic solution was added to the beaker in which aqueous solution (4) had been prepared, for complete absorption of aqueous solution (4). This was then dried for 4 hours under an air stream at 110°C to obtain acetic acid production catalyst 8.

### Comparative Example 1 (Production of acetic acid production catalyst 9 without gold addition)

Acetic acid production catalyst 9 was obtained by the same production process as Example 1, except that in the preparation of aqueous solution (1) in Example 1, 2.81 g of sodium tetrachloropalladate alone was used instead of 2.81 g of sodium tetrachloropalladate and 1.05 g of auric chloride.

### Comparative Example 2 (Production of acetic acid production catalyst 10 with low gold addition)

Acetic acid production catalyst 10 was obtained by the same production process as Example 1, except that in the preparation of aqueous solution (1) in Example 1, 0.01 g of auric chloride was used instead of 1.05 g of auric chloride.

### Comparative Example 3 (Production of acetic acid production catalyst 11 with high gold addition)

Acetic acid production catalyst 11 was obtained by the same production process as Example 1, except that in the preparation of aqueous solution (1) in Example 1, 15.17 g of auric chloride was used instead of 1.05 g of auric chloride.

Table 1 shows the amounts of each component charged for catalyst 1 to catalyst 11 in Examples 1-8 and Comparative Examples 1-3, expressed in terms of percent by weight.

**Table 1**

| Example | Catalyst component (a) | Catalyst component (b) | Catalyst component (c) | Catalyst component (d) | Catalyst component (e) |
|---|---|---|---|---|---|
| Catalyst 1 | 1.05 Pd | 0.53 Au | 21.7 (tungstosilicic acid) | | |
| Catalyst 2 | 1.05 Pd | 0.53 Au | 21.7 (tungstophosphoric acid) | | |
| Catalyst 3 | 1.05 Pd | 0.53 Au | 21.7 (lithium tungstosilicate) | | |
| Catalyst 4 | 1.05 Pd | 0.53 Au | 21.7 (tungstosilicic acid) | 0.071 (zinc) | |
| Catalyst 5 | 1.05 Pd | 0.53 Au | 21.7 (tungstosilicic acid) | 0.071 (chromium) | |
| Catalyst 6 | 1.05 Pd | 0.53 Au | 21.7 (tungstosilicic acid) | | 0.168 (tellurium) |
| Catalyst 7 | 1.05 Pd | 0.53 Au | 21.7 (tungstosilicic acid) | | 0.168 (bismuth) |
| Catalyst 8 | 1.05 Pd | 0.53 Au | 21.7 (tungstosilicic acid) | 0.071 (zinc) | 0.168 (tellurium) |
| Catalyst 9 | 1.05 Pd | | 21.7 (tungstosilicic acid) | | |
| Catalyst 10 | 1.05 Pd | 0.05 Au | 21.7 (tungstosilicic acid) | | |
| Catalyst 11 | 1.05 Pd | 8.04 Au | 21.7 (tungstosilicic acid) | | |

### Examples 9-16 and Comparative Examples 4-6 (Production of acetic acid)

A reaction tube (inner diameter: 25 mm) manufactured by SUS316 was packed with 18.5 g of each of the acetic acid production catalysts obtained in Examples 1-8 and Comparative Examples 1-3, and reaction was conducted with a catalyst bed reaction peak temperature of 200°C, a reaction pressure of 0.8 MPa (gauge pressure) and introduction of a gas supply comprising a mixture of ethylene, oxygen, steam and nitrogen at a volume ratio of 10:6:25:59 and a space velocity of 1800/h. The gas produced was cooled, and the condensed reaction solution that was collected was analyzed by gas chromatography (GC14B hydrogen flame detector by Shimazu Kagaku, KK.).

The reaction results are shown in Table 2.

**Table 2**

| | Catalyst used | Acetic acid space time yield (g/kg-catalyst·h) | Selectivity (%) | |
|---|---|---|---|---|
| | | | Acetic acid | Carbon dioxide gas |
| Example 9 | 1 | 215 | 87.4 | 6.3 |
| Example 10 | 2 | 211 | 86.3 | 7.0 |
| Example 11 | 3 | 208 | 89.5 | 5.2 |
| Example 12 | 4 | 245 | 88.5 | 5.7 |
| Example 13 | 5 | 240 | 89.2 | 5.5 |
| Example 14 | 6 | 258 | 91.2 | 5.0 |
| Example 15 | 7 | 238 | 90.5 | 4.9 |
| Example 16 | 8 | 288 | 91.2 | 5.7 |
| Comparative Example 4 | 9 | 186 | 76.3 | 21.3 |
| Comparative Example 5 | 10 | 188 | 80.2 | 15.5 |
| Comparative Example 6 | 11 | 196 | 82.0 | 16.3 |

### Example 17

After immersing 125 ml of a silica carrier (5 mmφ) in an aqueous solution containing 3.8 g of sodium tetrachloropalladate until absorption of the entire amount, it was added to 100 ml of an aqueous solution containing 7.6 g of sodium metasilicate and allowed to stand for 20 hours. Next, 5.6 g of hydrazine monohydrate was added to reduce the sodium tetrachloropalladate to metallic palladium, and after washing with water it was dried at 110°C for 4 hours. The carrier including the metallic palladium was then poured into an aqueous solution containing 20.6 g of tungstosilicic acid and 0.57 g of chromium nitrate · 9H₂O and after absorption of the entire solution, it was dried at 110°C for 4 hours.

A reaction tube was packed with 22.5 ml of the obtained catalyst, and a mixed gas of ethylene:oxygen:steam:nitrogen at a volume ratio of 10:6:25:59 was introduced at a flow rate of 45 N liters/H for reaction at a temperature of 200°C and a pressure of 0.8 MPa (gauge pressure).

The resulting gas was cooled and the condensed reaction solution collected was analyzed by gas chromatography.

### Example 18

The same procedure was followed as in Example 17, except that 0.62 g of manganese nitrate·6H₂O was used instead of the chromium nitrate · 9H₂O in Example 17.

### Example 19

The same procedure was followed as in Example 17, except that 0.63 g of cobalt nitrate · 6H₂O was used instead of the chromium nitrate · 9H₂O in Example 17.

### Example 20

The same procedure was followed as in Example 17, except that 0.63 g of nickel nitrate · 6H₂O was used instead of the chromium nitrate · 9H₂O in Example 17.

### Comparative Example 7

The same procedure was followed as in Example 17, except that 0.30 g of lithium nitrate was used instead of the chromium nitrate·9H₂O in Example 17.

### Comparative Example 8

The same procedure was followed as in Example 17, except that 0.52 g of copper nitrate · 3H₂O was used instead of the chromium nitrate · 9H₂O in Example 17.

The reaction results for Examples 17-20 and Comparative Examples 7 and 8 are shown in Table 3.

**Table 3**

| | Heteropoly acid salt neutralizing element | Acetic acid space time yield (g/l·H) | Selectivity (%) | |
|---|---|---|---|---|
| | | | Acetic acid | Carbon dioxide |
| Example 17 | Cr | 95 | 65.3 | 20.9 |
| Example 18 | Mn | 93 | 65.7 | 19.7 |
| Example 19 | Co | 96 | 64.9 | 22.3 |
| Example 20 | Ni | 98 | 63.7 | 22.7 |
| Comp. Ex. 7 | Li | 83 | 63.6 | 23.5 |
| Comp. Ex. 8 | Cu | 77 | 61.9 | 25.2 |

### Example 21

After immersing 125 ml of a silica carrier (5 mmφ) in an aqueous solution containing 3.8 g of sodium tetrachloropalladate until absorption of the entire amount, it was added to 100 ml of an aqueous solution containing 7.6 g of sodium metasilicate and allowed to stand for 20 hours. Next, 5.6 g of hydrazine monohydrate was added to reduce the sodium tetrachloropalladate to metallic palladium, and after washing with water it was dried at 110°C for 4 hours. The carrier including the metallic palladium was then poured into an aqueous solution containing 0.52 g of potassium tellurite, and after absorption of the entire solution, it was dried at 110°C for 4 hours. Next, the carrier including the metallic palladium and tellurium was poured into an aqueous solution containing 20.6 g of tungstosilicic acid and 0.57 g of chromium nitrate · 9H₂O, and after absorption of the entire solution, it was dried at 110°C for 4 hours.

A reaction tube was packed with 22.5 ml of the obtained catalyst, and a mixed gas of ethylene:oxygen:steam:nitrogen at a volume ratio of 10:6:25:59 was introduced at a flow rate of 45 N liters/H for reaction at a temperature of 200°C and a pressure of 0.8 MPa (gauge pressure).

The resulting gas was cooled and the condensed reaction solution collected was analyzed by gas chromatography.

### Example 22

The same procedure was followed as in Example 21, except that 0.62 g of manganese nitrate · 9H₂O was used instead of the chromium nitrate · 9H₂O in Example 21.

### Example 23

The same procedure was followed as in Example 21, except that 0.63 g of cobalt nitrate · 6H₂O was used instead of the chromium nitrate · 9E₂O in Example 21.

### Example 24

The same procedure was followed as in Example 21, except that 0.63 g of nickel nitrate · 6H₂O was used instead of the chromium nitrate · 9H₂O in Example 21.

### Comparative Example 9

The same procedure was followed as in Example 21, except that 0.30 g of lithium nitrate was used instead of the chromium nitrate · 9H₂O in Example 21.

### Comparative Example 10

The same procedure was followed as in Example 21, except that 0.52 g of copper nitrate · 3H₂O was used instead of the chromium nitrate · 9H₂O in Example 21.

The reaction results for Examples 21-24 and Comparative Examples 9 and 10 are shown in Table 4.

**Table 4**

| | Heteropoly acid salt neutralizing element | Group (c) element | Acetic acid space time yield g/l·H | Selectivity (%) | |
|---|---|---|---|---|---|
| | | | | Acetic acid | Carbon dioxide |
| Example 21 | Cr | Te | 178 | 90.7 | 6.06 |
| Example 22 | Mn | Te | 178 | 90.9 | 5.66 |
| Example 23 | Co | Te | 177 | 91.0 | 5.94 |
| Example 24 | Ni | Te | 180 | 90.1 | 6.42 |
| Comp.Ex. 9 | Li | Te | 156 | 89.6 | 6.66 |
| Comp.Ex. 10 | Cu | Te | 140 | 87.0 | 7.56 |

### Industrial Applicability

According to the present invention, it is possible to achieve higher space time yields, lower carbon dioxide selectivity and less catalyst deterioration than with conventional catalysts, thereby allowing production of acetic acid with higher productivity.

## Claims

1. A catalyst for production of acetic acid, which is a catalyst used in a process for production of acetic acid that comprises reacting ethylene and oxygen in a gas phase and having (a) metallic palladium, (b) metallic gold and (c) at least one compound selected from among heteropoly acids and/or salts thereof loaded on a carrier, wherein the weight ratio of the (a) metallic palladium and (b) metallic gold is in the range of (a) metallic palladium:(b) metallic gold = 1:0.1-5.0.

2. A catalyst according to claim 1, wherein (d) at least one element selected from the group consisting of Group 6 elements, Group 7 elements, Group 8 elements, Group 9 elements, Group 10 elements and Group 12 elements of the Periodic Table is also loaded on the carrier.

3. A catalyst according to claim 1 or 2, wherein (e) at least one element selected from the group consisting of Group 11 elements, Group 14 elements, Group 15 elements and Group 16 elements of the Periodic Table is also loaded on the carrier.

4. A process for production of a catalyst for production of acetic acid according to claim 1, which comprises the following steps 1 and 2.
Step 1
A step wherein the (a) metallic palladium and (b) metallic gold are loaded on a carrier to obtain a metallic palladium-loaded catalyst.
Step 2
A step wherein the (c) at least one compound selected from among heteropoly acids and/or salts thereof is loaded on the metallic palladium-loaded catalyst obtained in step 1 to obtain a catalyst for production of acetic acid.

5. A process for production of a catalyst for production of acetic acid according to claim 2, which comprises the following steps 1 and 2.
Step 1
A step wherein the (a) metallic palladium, (b) metallic gold and (d) at least one element selected from the group consisting of Group 6 elements, Group 7 elements, Group 8 elements, Group 9 elements, Group 10 elements and Group 12 elements of the Periodic Table are loaded on a carrier to obtain a metallic palladium-loaded catalyst.
Step 2
A step wherein the (c) at least one compound selected from among heteropoly acids and/or salts thereof is loaded on the metallic palladium-loaded catalyst obtained in step 1 to obtain a catalyst for production of acetic acid.

6. A process for production of a catalyst for production of acetic acid according to claim 3, which comprises the following steps 1 and 2.
Step 1
A step wherein the (a) metallic palladium, (b) metallic gold and (e) at least one element selected from the group consisting of Group 11 elements, Group 14 elements, Group 15 elements and Group 16 elements of the Periodic Table are loaded on a carrier to obtain a metallic palladium-loaded catalyst.
Step 2
A step wherein the (c) at least one compound selected from among heteropoly acids and/or salts thereof is loaded on the metallic palladium-loaded catalyst obtained in step 1 to obtain a catalyst for production of acetic acid.

7. A process for production of a catalyst for production of acetic acid according to claim 3, which comprises the following steps 1 and 2.
Step 1
A step wherein the (a) metallic palladium, (b) metallic gold, (d) at least one element selected from the group consisting of Group 6 elements, Group 7 elements, Group 8 elements, Group 9 elements, Group 10 elements and Group 12 elements of the Periodic Table and (e) at least one element selected from the group consisting of Group 11 elements, Group 14 elements, Group 15 elements and Group 16 elements of the Periodic Table are loaded on a carrier to obtain a metallic palladium-loaded catalyst.
Step 2
A step wherein the (c) at least one compound selected from among heteropoly acids and/or salts thereof is loaded on the metallic palladium-loaded catalyst obtained in step 1 to obtain a catalyst for production of acetic acid.

8. A process for production of acetic acid which comprises reacting ethylene and oxygen in a gas phase in the presence of a catalyst for production of acetic acid according to any one of claims 1 to 3.

9. A process for production of acetic acid which comprises reacting ethylene and oxygen in a gas phase in the presence of water, and in the presence of a catalyst for production of acetic acid according to any one of claims 1 to 3.

10. A catalyst for production of acetic acid, which is a catalyst that is used in a process for production of acetic acid by reacting ethylene and oxygen and contains (a) metallic palladium and (b) a heteropoly acid salt, wherein the (b) heteropoly acid salt comprises a metal salt wherein all or a portion of the hydrogen atoms of the heteropoly acid are replaced with at least one element selected from the group consisting of Group 6 elements, Group 7 elements, Group 9 elements and Group 10 elements of the Periodic Table.

11. A catalyst for production of acetic acid according to claim 10, wherein the (a) metallic palladium and (b) heteropoly acid salt are held on a carrier.

12. A catalyst for production of acetic acid, which is a catalyst that is used in a process for production of acetic acid by reacting ethylene and oxygen and contains (a) metallic palladium, (b) a heteropoly acid salt and (c) at least one element selected from the group consisting of Group 8 elements, Group 11 elements, Group 12 elements, Group 14 elements, Group 15 elements and Group 16 elements of the Periodic Table, wherein the (b) heteropoly acid salt comprises a metal salt wherein all or a portion of the hydrogen atoms of the heteropoly acid are replaced with at least one element selected from the group consisting of Group 6 elements, Group 7 elements, Group 9 elements and Group 10 elements of the Periodic Table.

13. A catalyst for production of acetic acid according to claim 12, wherein the (a) metallic palladium, (b) heteropoly acid salt and (c) at least one element selected from the group consisting of Group 8 elements, Group 11 elements, Group 12 elements, Group 14 elements, Group 15 elements and Group 16 elements of the Periodic Table are held on a carrier.

14. A catalyst for production of acetic acid according to any one of claims 10 to 13, wherein the elements replacing all or a portion of the hydrogen atoms of the heteropoly acid consist of at least one element selected from the group consisting of chromium, manganese, cobalt and nickel.

15. A process for production of a catalyst for production of acetic acid according to claim 10, which comprises the following steps 1 and 2.
Step 1
A step wherein a suspension of the (a) metallic palladium is obtained.
Step 2
A step wherein the (b) heteropoly acid salt is dissolved or suspended in the metallic palladium suspension obtained in step 1, and the solvent is removed to obtain a catalyst for production of acetic acid.

16. A process for production of a catalyst for production of acetic acid according to claim 11, which comprises the following steps 1 and 2.
Step 1
A step wherein the (a) metallic palladium is loaded on a carrier to obtain a metallic palladium-loaded catalyst.
Step 2
A step wherein the (b) heteropoly acid salt is loaded on the metallic palladium-loaded catalyst obtained in step 1 to obtain a catalyst for production of acetic acid.

17. A process for production of a catalyst for production of acetic acid according to claim 12 which comprises the following steps 1 and 2.
Step 1
A step wherein a suspension containing the (a) metallic palladium and (c) at least one element selected from the group consisting of Group 8 elements, Group 11 elements, Group 12 elements, Group 14 elements, Group 15 elements and Group 16 elements of the Periodic Table is obtained.
Step 2
A step wherein the (b) heteropoly acid salt is dissolved or suspended in the metallic palladium suspension obtained in step 1, and the solvent is removed to obtain a catalyst for production of acetic acid.

18. A process for production of a catalyst for production of acetic acid according to claim 12, which comprises the following steps 1 and 2.
Step 1
A step wherein a suspension of the (a) metallic palladium is obtained.
Step 2
A step wherein the (b) heteropoly acid salt and (c) at least one element selected from the group consisting of Group 8 elements, Group 11 elements, Group 12 elements, Group 14 elements, Group 15 elements and Group 16 elements of the Periodic Table, and/or at least one compound from among compounds containing these elements, is dissolved or suspended in the metallic palladium suspension obtained in step 1, and the solvent is removed to obtain a catalyst for production of acetic acid.

19. A process for production of a catalyst for production of acetic acid according to claim 12, which comprises the following steps 1 and 2.
Step 1
A step wherein a suspension containing the (a) metallic palladium and (c) at least one element selected from the group consisting of Group 8 elements, Group 11 elements, Group 12 elements, Group 14 elements, Group 15 elements and Group 16 elements of the Periodic Table is obtained.
Step 2
A step wherein the (b) heteropoly acid salt and (c) at least one element selected from the group consisting of Group 8 elements, Group 11 elements, Group 12 elements, Group 14 elements, Group 15 elements and Group 16 elements of the Periodic Table, and/or at least one compound from among compounds containing these elements, is dissolved or suspended in the metallic palladium suspension obtained in step 1, and the solvent is removed to obtain a catalyst for production of acetic acid.

20. A process for production of a catalyst for production of acetic acid according to claim 13, which comprises the following steps 1 and 2.
Step 1
A step wherein the (a) metallic palladium and (c) at least one element selected from the group consisting of Group 8 elements, Group 11 elements, Group 12 elements, Group 14 elements, Group 15 elements and Group 16 elements of the Periodic Table are loaded on a carrier to obtain a metallic palladium-loaded catalyst.
Step 2
A step wherein the (b) heteropoly acid salt is loaded on the metallic palladium-loaded catalyst obtained in step 1 to obtain a catalyst for production of acetic acid.

21. A process for production of a catalyst for production of acetic acid according to claim 13, which comprises the following steps 1 and 2.
Step 1
A step wherein the (a) metallic palladium is loaded on a carrier to obtain a metallic palladium-loaded catalyst.
Step 2
A step wherein the (b) heteropoly acid salt and (c) at least one element selected from the group consisting of Group 8 elements, Group 11 elements, Group 12 elements, Group 14 elements, Group 15 elements and Group 16 elements of the Periodic Table are loaded on the metallic palladium-loaded catalyst obtained in step 1 to obtain a catalyst for production of acetic acid.

22. A process for production of a catalyst for production of acetic acid according to claim 13, which comprises the following steps 1 and 2.
Step 1
A step wherein the (a) metallic palladium and (c) at least one element selected from the group consisting of Group 8 elements, Group 11 elements, Group 12 elements, Group 14 elements, Group 15 elements and Group 16 elements of the Periodic Table are loaded on a carrier to obtain a metallic palladium-loaded catalyst.
Step 2
A step wherein the (b) heteropoly acid salt and (c) at least one element selected from the group consisting of Group 8 elements, Group 11 elements, Group 12 elements, Group 14 elements, Group 15 elements and Group 16 elements of the Periodic Table are loaded on the metallic palladium-loaded catalyst obtained in step 1 to obtain a catalyst for production of acetic acid.

23. A process for production of acetic acid which comprises reacting ethylene and oxygen in a gas phase in the presence of a catalyst for production of acetic acid according to any one of claims 10 to 14.

24. A process for production of acetic acid which comprises reacting ethylene and oxygen in a gas phase in the presence of water, and in the presence of a catalyst for production of acetic acid according to any one of claims 10 to 14.
